# EUROPEAN PATENT SPECIFICATION

(11) **EP 1 326 544 B1**
(45) Date of publication and mention of the grant of the patent: **18.08.2004**
(21) Application number: 00982664.5
(22) Date of filing: 17.10.2000
(51) Int. Cl.: A61B 17/122

(54) **ANEURYSM CLIP**
ANEURYSMAKLEMME
CLIP D'ANEVRISME

(43) Date of publication of application: 16.07.2003
(73) Proprietor: Aesculap AG & Co. KG, 78532 Tuttlingen (DE)
(72) Inventor: LUTZE, Theodor, 78582 Balgheim (DE); FISCHER, Manfred, 78532 Tuttlingen (DE); KOPITNIK, Thomas, A., Jr., Dallas, TX 75235 (US)
(74) Representative: HOEGER, STELLRECHT & PARTNER Patentanwälte
(86) International application number: PCT/US2000/041200
(87) International publication number: WO 2002/032327

(56) References cited:
- EP-A- 0 346 084
- WO-A-98/18389
- DE-A- 3 302 707
- US-A- 3 802 437
- US-A- 5 019 092

## Description

The invention relates to an aneurysm clip with two arms which are resiliently pivotable towards each other and carry at their free end clamping bars which are resiliently clamped against each other by the spring force of the arms, with outwardly curved sections of the arms adjoining the clamping bars and jointly forming a receiving space for a vessel.

Such aneurysm clips are known, for example, from EP 0 346 084 A1 or from US-A-3,802,437.

In the clips of US-A-3,802,437 outwardly curved sections whose width corresponds to the length of the clamping bars adjoin the relatively long clamping bars so as to produce a sleeve-shaped receiving space for a vessel. When several such aneurysm clips are applied alongside one another to a vessel, these outwardly curved, sleeve-shaped sections may impede one another and so it is not possible to place these clips in the desired manner close to one another.

In the aneurysm clip of EP 0 346 084 the clamping bars are bent laterally out of the center plane of the aneurysm clip to produce an asymmetrical arrangement, which involves the danger of the clips sliding down at one side. The one-sided stress on the clips by the bent clamping bars can result in canting and wedging, which can put the safety of the application of such an aneurysm clip at risk.

The object of the invention is to so improve a generic aneurysm clip that it enables, on the one hand, an unimpeded application, also of a plurality of clips, to a vessel, and, on the other hand, a particularly reliable sealing-off in the area of the clamping bars.

This object is accomplished with an aneurysm clip of the kind described at the outset, in accordance with the invention, in that the clamping bars protrude from the arms towards both sides and thereby project laterally on both sides over the width of the arms in the outwardly curved sections.

Such an aneurysm clip thus has relatively narrow arms in the area of the outwardly curved sections, but clamping bars which extend essentially transversely to the center plane of the aneurysm clip and project towards either side over the width of the arms are arranged at the end of the arms. The clamping bars are, therefore, supported on either side of the center plane of the aneurysm clip, and, at the same time, it is possible to place several aneurysm clips closely alongside one another because the arms are narrower than the clamping bars in the area of the outwardly curved sections surrounding the vessel. Therefore, several clips can be applied in such a way that their clamping bars overlap at the sides, while the arms are placed closely alongside one another in the outwardly curved sections. It is thus possible for the operator to apply several aneurysm clips to a vessel in such a way that a dividing line is ensured, possibly several times, and owing to the protruding of the clamping bars from the arms on either side, the aneurysm clips are optimally secured against canting and undesired twisting and therefore against sliding down.

Provision is made in a preferred embodiment for the clamping bars to be of straight-lined design in their longitudinal direction.

It is, however, also possible for the clamping bars to be bent in their longitudinal direction, and the arms can be arranged on the convex side of the clamping bars or on the concave side of the clamping bars so that the clamping bars are bent either away from the arms or towards the arms.

It is particularly advantageous for the clamping bars to be of symmetrical design in relation to the center plane of the aneurysm clip. The aneurysm clip can then be applied in the same way in both directions, and, in addition, the forces exerted on the clip by the symmetrical design are equal, which reliably prevents tilting and sliding down.

In particular, provision may be made for the clamping bars to extend in the area of connection to the arms at an angle of 90° to the center plane of the aneurysm clip.

However, in a special embodiment provision may also be made for the clamping bars to extend in the area of connection to the arms at an incline to the center plane of the aneurysm clip so that it is possible to arrange the arms such that they are at an incline in relation to the clamping line defined by the clamping bars.

In particular, the outwardly curved section can be of substantially circular design.

It is, however, also possible for the outwardly curved section to be oval design. This is expedient, particularly when the clamping bars extend at an incline to the center plane of the aneurysm clip because the outwardly curved section can then surround a vessel of substantially circular cross section when the arms also extend at an incline relative to the vessel.

The clamping bars can have a length which is between twice and eight times the width of the arms in the outwardly curved section. Clamping bars of different length can also be united in a set of such aneurysm clips.

The aneurysm clip can be of such design that the two arms are joined to each other by a resilient connecting section, for example, a single or multiple winding of a spring wire which continues in the two arms.

In another embodiment provision may be made for the two arms to be pivotally connected to each other and clamped against each other by a separate spring. In particular, this spring itself can form a bearing shaft for the pivot bearing of the two arms.

The arms connected to the clamping bars can have at least one opening in the area adjoining the clamping bars so that the arms are divided into two webs, each of which is connected to the clamping bars.

In particular, in this embodiment provision may be made for the arms in this area to be wider so that the webs extend in the shape of a V. Such a widening of the arms is also possible without provision of an opening.

The following description of preferred embodiments of the invention serves in conjunction with the drawings to explain the invention in greater detail. The drawings show:
- Figure 1: a side view of a first preferred embodiment of an aneurysm clip applied to a vessel, and a straight-lined clamping bar which in comparison with the width of the clamping arms is long;
- Figure 2: a sectional view taken along 2-2 in Figure 1;
- Figure 3: a view similar to Figure 1 with three aneurysm clips applied alongside one another with short, straight-lined clamping bars and a resilient connecting section;
- Figure 4: a sectional view taken along 4-4 in Figure 3;
- Figure 5: a view similar to Figure 4 with aneurysm clips with clamping bars of different lengths applied in offset relation to one another;
- Figure 6: a view similar to Figure 3 with two aneurysm clips with short, straight-lined clamping bars and an aneurysm clip with straight-lined clamping bars extending at an incline;
- Figure 7: a view similar to Figure 3 with a single aneurysm clip with clamping bars bent away from the arms;
- Figure 8: a view similar to Figure 7 with clamping bars bent towards the arms; and
- Figure 9: a view of a modified embodiment of an aneurysm clip similar to that of Figure 5 with arms which widen in the shape of a V and have an opening.

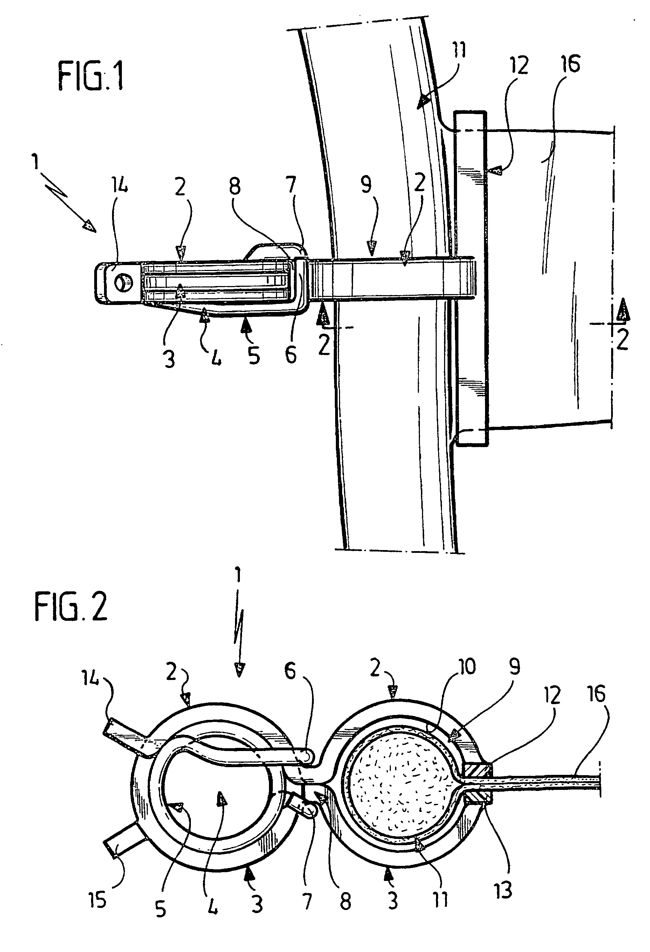

The aneurysm clip 1 shown in Figures 1 and 2 comprises two arms 2, 3 made of a band-shaped material of rectangular cross section. At their rear end, both arms are bent in the shape of a circular arc and thus form a substantially circular bearing section 4 in which a helical spring 5 essentially consisting of two wound layers is located. This helical spring 5 abuts on the inner sides of the bearing section 4 and forms a bearing shaft which joins the two arms 2 and 3 in the area of the bearing section 4 for pivotal movement about the axis of the bearing section 4. The free ends 6, 7 of the helical spring 5 are supported on the outer side of the two arms 2, 3 immediately adjacent to the bearing section 4 on the latter and pivot the two arms 2 and 3 towards each other in such a way that they can only be pivoted apart elastically against the force of the helical spring 5.

Adjoining the bearing section 4 is a short intermediate section 8 in which the arms 2 and 3 extend parallel to each other and essentially abut on one another when the clip is closed.

This intermediate section 8 passes into a front vessel section 9 in which the two arms 2, 3 are outwardly bent in the shape of a semicircle and thus enclose a substantially circular receiving space 10 which receives a vessel 11 when the aneurysm clip is applied.

At their free ends, both arms 2, 3 are connected to straight-lined clamping bars which in the embodiment shown in Figures 1 and 2 extend parallel to the pivot axis of the two arms 2, 3, i.e., protrude vertically from the center plane of the aneurysm clip 1 towards both sides, more particularly, in the same way towards both sides, i.e., in the area of the clamping bars 12, 13 the aneurysm clip is of symmetrical design in relation to its center plane. The clamping bars 12, 13 are located directly opposite each other and are pressed against each other by the helical spring 5 in the closed state such that they abut on one another throughout their entire length.

In the embodiment shown in Figures 1 and 2, the length of the clamping bars 12, 13 in their longitudinal direction is approximately eight times greater than the width of the arms 2, 3 in the area of the receiving space 10.

To apply the aneurysm clip 1 illustrated in Figures 1 and 2, the arms 2 and 3 are pivoted outwards against the force of the helical spring 5, for example, by pressure on projections 14, 15 protruding radially at the rear ends of the arms 2, 3 so that the clamping bars 12 and 13 can be guided past a vessel 11 until the vessel 11 is located in the area of the receiving space 10 in the vessel section 9. When closing the aneurysm clip 1, the arms 2 and 3 are pivoted towards each other under the action of the helical spring 5, and the clamping bars 12, 13 position themselves at vessel walls 16 of a protuberance or an aneurysm protruding at the sides from the vessel 11 and thus close off this protuberance towards the vessel 11 over the entire width thereof. One aneurysm clip 1 of the described kind is thus sufficient to close off a protuberance over quite a large area in essentially the shape of a line. Owing to the relatively large length of the clamping bars 12, 13, this length is substantially greater than the width of the arms 2, 3 and thus also the width of the aneurysm clip 1 in a direction perpendicular to the center plane.

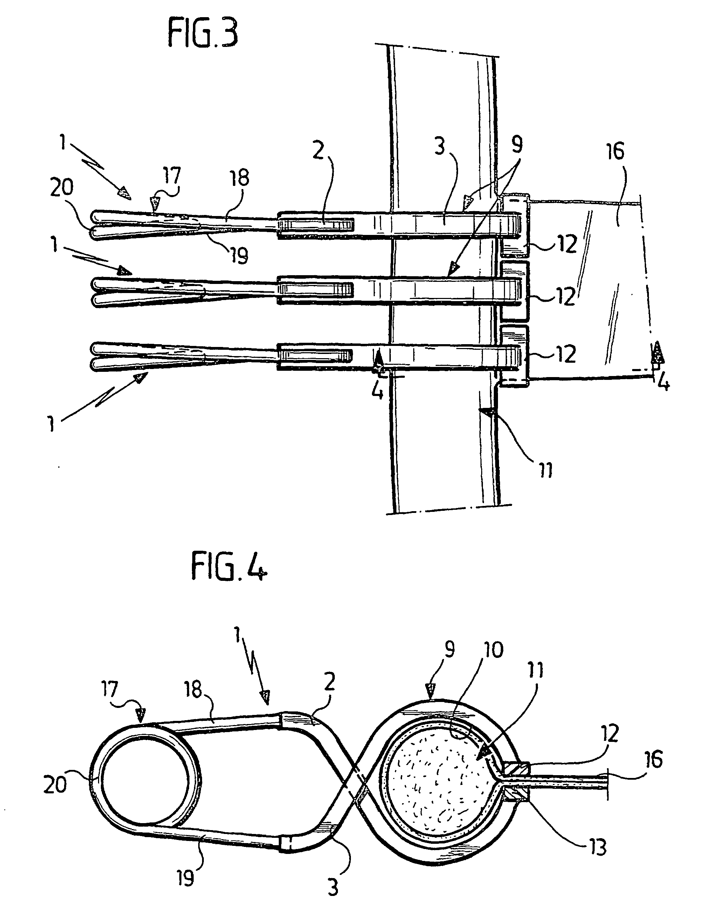

A similar arrangement is shown in the embodiment of Figures 3 and 4, and, therefore, corresponding parts bear the same reference numerals.

Differently from the embodiment of Figures 1 and 2, the arms 2 and 3 of this embodiment are not pivotally joined to each other in the area of a bearing section 4 by a helical spring 5, but instead the two arms 2 and 3 are joined to each other by an elastic connecting section 17 having two legs 18 and 19, which are each joined to one of the arms 2 and 3, respectively, and are joined together by an elastic web 20 so that the legs 18 and 19 are elastically bendable apart owing to the inherent elasticity of the web. In the area of the web 20, the material of the connecting section 17 can be formed through 360° on itself in the form of an eyelet or helix so as to produce an elastic coil by means of which the inherent elasticity in the area of the web 20 can be increased. It is preferable for the arms 2 and 3 and the connecting section 17 to be integrally made from an elastic material, for example, from spring wire, but it is also possible for the connecting section 17 to be joined to arms made of a different material, for example, the arms 2, 3 can consist of a sterilizable plastic material and be joined to legs 18 and 19 made of spring wire.

In the embodiment of Figures 3 and 4, the arms 2 and 3 also form a vessel section 9 with clamping bars 12, 13 arranged at the front end thereof. However, in the embodiment of Figures 3 and 4 these are significantly shorter than in the embodiment of Figure 1, the length of the clamping bars in this embodiment being only approximately three times the width of the arms 2, 3.

It is thereby possible to arrange several such aneurysm clips 1 directly alongside one another on a vessel 11 in the area of the vessel wall 16 bulging out at the side so that a line-shaped clamping is also achieved, but using three aneurysm clips 1 and thus employing a higher clamping force since each aneurysm clip 1 exerts its inherent clamping force on a shorter clamping bar 12, 13.

It is, of course, also possible to use aneurysm clips 1 with correspondingly short clamping bars, which like the aneurysm clip 1 of Figures 1 and 2 do not have an elastic connecting section 17 at their disposal, but a bearing section 4 with a helical spring 5.

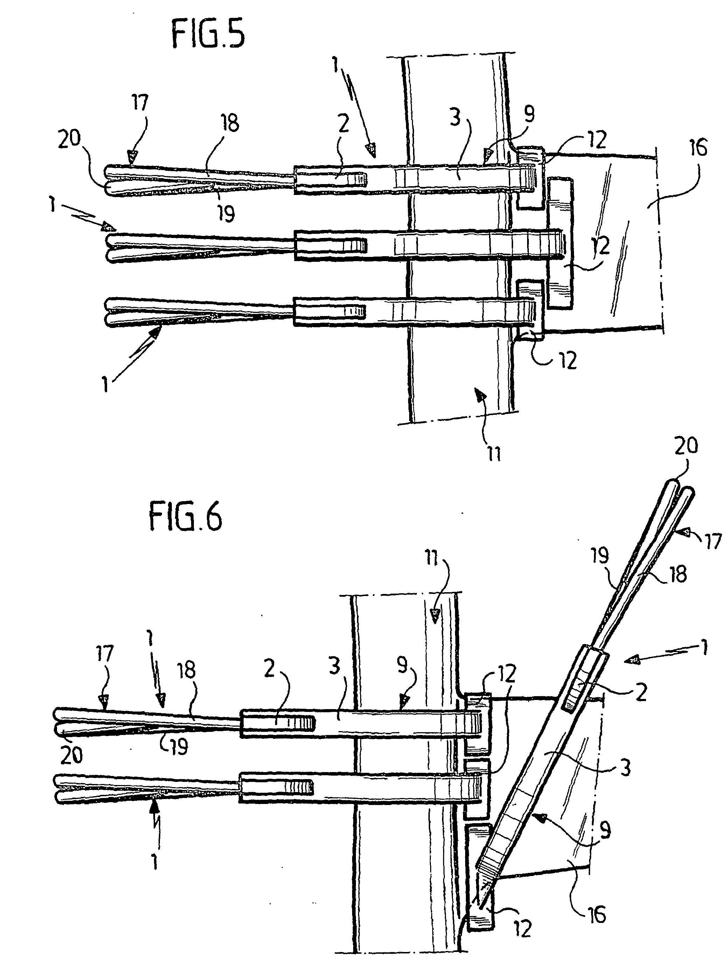

Aneurysm clips 1 which are of essentially the same construction as in the embodiment of Figure 3 are used in the embodiment of Figure 5. Aneurysm clips whose clamping bars 12, 13 are of different length are used at only one point of application. In addition, the dimensions of the aneurysm clips 1 and 2 in the longitudinal direction are selected so as to differ, and so, for example, the center aneurysm clip 1 of Figure 5 is of somewhat longer construction than the two aneurysm clips 1 at the sides. The clamping bars 12, 13 of the three aneurysm clips 1 can thus be arranged at a different spacing from the vessel 11. An overlapping of neighboring clamping bars 12, 13 is, therefore, possible, and a particularly effective closure of the vessel wall 16 of a lateral protuberance is thereby achieved. The different length is, for example, achievable by the vessel section 9 in the case of the longer aneurysm clip 1 not - as in the case of the other aneurysm clips 1 - surrounding a circular area, but an oval area which is elongate in the longitudinal direction.

The aneurysm clips shown in Figure 6 correspond to those of Figure 3, and corresponding parts bear the same reference numerals.

In the embodiment of Figure 6, one of the three aneurysm clips arranged alongside one another is replaced by an aneurysm clip on which the clamping bars 12, 13 extend at an incline to the longitudinal direction of the arms 2, 3, i.e., at an incline to the center plane of the aneurysm clip 1. Also, in this embodiment of the aneurysm clip 1 an oval vessel section 9 is likewise effected, with its long axis running in the longitudinal direction of the aneurysm clip 1.

It is thus possible to apply this aneurysm clip 1 to the vessel 11, also from the side opposite the vessel 11, such that the vessel wall 16 is clamped off in continuation of the clamping bars 12, 13 of the two other aneurysm clips 1, with this aneurysm clip 1 then not surrounding the vessel 11, but the vessel wall 16 of the protuberance.

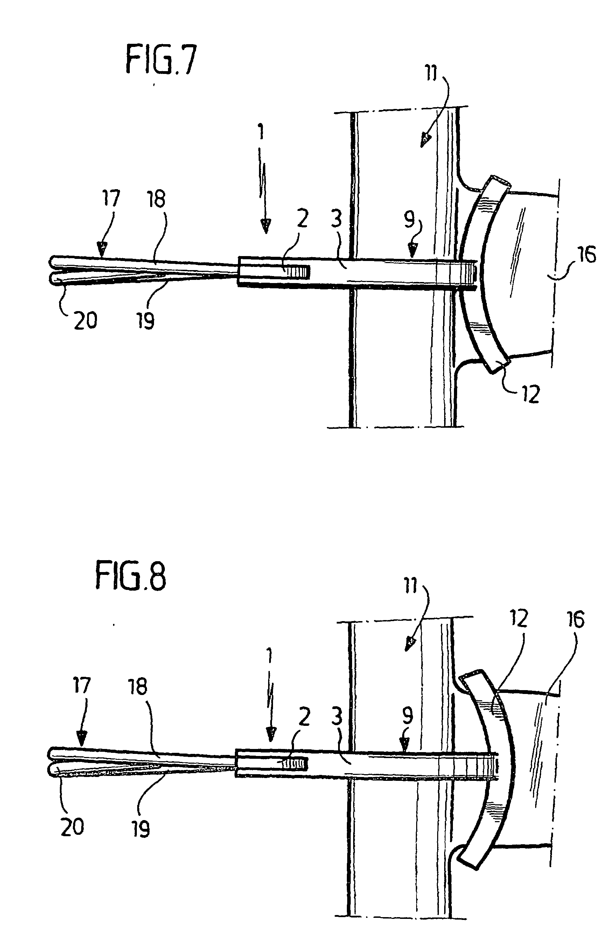

The aneurysm clips of Figures 7 and 8 correspond fully to those of Figure 3, with the difference that the clamping bars 12, 13 are not of straight-lined construction, but have the shape of a circular arc. In the embodiment of Figure 7 the arms 2, 3 are located on the convex side of the arcuate clamping bars 12, 13, in the embodiment of Figure 8 on the concave side. In both cases, the construction of the clamping bars 12, 13 is symmetrical in relation to the center plane, and clamping bars 12, 13 located opposite each other are, of course, of the same design so that the clamping surfaces of these clamping bars 12, 13 lie opposite each other throughout their entire surface during the closing.

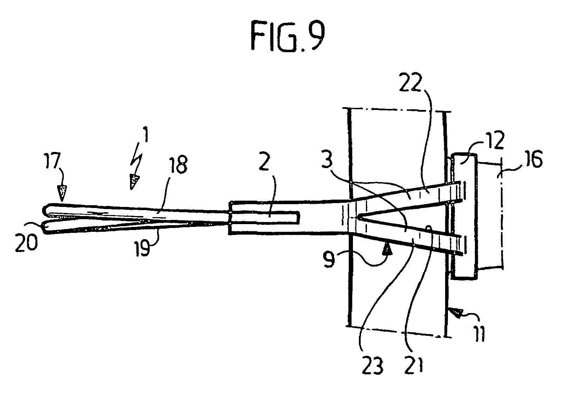

A further preferred embodiment of an aneurysm clip which corresponds substantially to the constructions shown in Figure 5 is shown in Figure 9. In this embodiment, the arms 2, 3 are widened in the area adjoining the clamping bars 12, 13 and have an opening 21 so that the arms 2, 3 extend in the form of two webs 22 and 23 arranged in the shape of a V and opening into the clamping bars 12, 13.

## Claims

1. Aneurysm clip (1) with two arms (2, 3) which are resiliently pivotable towards each other and carry at their free end clamping bars (12, 13) which are resiliently clamped against each other by the spring force of the arms (2, 3), with outwardly curved sections (9) of the arms (2, 3) adjoining the clamping bars (12, 13) and jointly forming a receiving space (10) for a vessel (11), **characterized in that** the clamping bars (12, 13) protrude from the arms (2, 3) towards both sides and thereby project laterally on both sides over the width of the arms (2, 3) in the outwardly curved sections (9).

2. Aneurysm clip as defined in claim 1, **characterized in that** the clamping bars (12, 13) are of straight-lined design in their longitudinal direction.

3. Aneurysm clip as defined in claim 1, **characterized in that** the clamping bars (12, 13) are bent in their longitudinal direction.

4. Aneurysm clip as defined in claim 3, **characterized in that** the arms (2, 3) are arranged on the convex side of the clamping bars (12, 13).

5. Aneurysm clip as defined in claim 3, **characterized in that** the arms (2, 3) are arranged on the concave side of the clamping bars (12, 13).

6. Aneurysm clip as defined in any one of the preceding claims, **characterized in that** the clamping bars (12, 13) are of symmetrical design in relation to the center plane of the aneurysm clip (1).

7. Aneurysm clip as defined in any one of the preceding claims, **characterized in that** in the area of connection to the arms (2, 3) the clamping bars (12, 13) extend at an angle of 90° to the center plane of the aneurysm clip (1).

8. Aneurysm clip as defined in any one of claims 1 to 5, **characterized in that** in the area of connection to the arms (2, 3) the clamping bars (12, 13) extend at an incline to the center plane of the aneurysm clip (1).

9. Aneurysm clip as defined in any one of the preceding claims, **characterized in that** the outwardly curved section (9) is of substantially circular design.

10. Aneurysm clip as defined in any one of claims 1 to 8, **characterized in that** the outwardly curved section (9) is of oval design.

11. Aneurysm clip as defined in any one of the preceding claims, **characterized in that** the clamping bars (12, 13) have a length which is between twice and ten times the width of the arms (2, 3) in the outwardly curved section (9).

12. Aneurysm clip as defined in any one of the preceding claims, **characterized in that** the two arms (2, 3) are joined to each other by a resilient connecting section (17).

13. Aneurysm clip as defined in any one of claims 1 to 11, **characterized in that** the two arms (2, 3) are pivotally connected to each other and are clamped against each other by a separate spring (5).

14. Aneurysm clip as defined in claim 13, **characterized in that** the spring (5) itself forms a bearing shaft for the pivot bearing of the two arms (2, 3).

15. Aneurysm clip as defined in any one of the preceding claims, **characterized in that** the pivotable arms (2, 3) have an opening (21) in the area in which they adjoin the clamping bars (12, 13).

16. Aneurysm clip as defined in any one of the preceding claims, **characterized in that** the arms (2, 3) widen towards the clamping bars (12, 13) in the area in which they adjoin the clamping bars (12, 13).

## Patentansprüche

1. Aneurysmenclip (1) mit zwei federnd gegeneinander verschwenkbaren Armen (2, 3), die an ihrem freien Ende Klemmleisten (12, 13) tragen, die durch die Federkraft der Arme (2, 3) federnd gegeneinander gespannt werden und an die sich augebauchte, gemeinsam einen Aufnahmeraum (10) für ein Gefäß (11) bildende Abschnitte (9) der Arme (2, 3) anschließen, **dadurch gekennzeichnet, daß** die Klemmleisten (12, 13) nach beiden Seiten von den Armen (2, 3) abstehen und dadurch seitlich auf beiden Seiten über die Breite der Arme (2, 3) in den ausgebauchten Abschnitten (9) überstehen.

2. Aneurysmenclip nach Anspruch 1, **dadurch gekennzeichnet, daß** die Klemmleisten (12, 13) in ihrer Längsrichtung geradlinig ausgebildet sind.

3. Aneurysmenclip nach Anspruch 1, **dadurch gekennzeichnet, daß** die Klemmleisten (12, 13) in ihrer Längsrichtung gebogen sind.

4. Aneurysmenclip nach Anspruch 3, **dadurch gekennzeichnet, daß** die Arme (2, 3) an der konvexen Seite der Klemmleisten (12, 13) angeordnet sind.

5. Aneurysmenclip nach Anspruch 3, **dadurch gekennzeichnet, daß** die Arme (2, 3) an der konkaven Seite der Klemmleisten (12, 13) angeordnet sind.

6. Aneurysmenclip nach einem der voranstehenden Ansprüche, **dadurch gekennzeichnet, daß** die Klemmleisten (12, 13) symmetrisch zu der Mittelebene des Aneurysmenclips (1) ausgebildet sind.

7. Aneurysmenclip nach einem der voranstehenden Ansprüche, **dadurch gekennzeichnet, daß** die Klemmleisten (12, 13) im Verbindungsbereich zu den Armen (2, 3) unter einem Winkel von 90° zur Mittelebene des Aneurysmenclips (1) verlaufen.

8. Aneurysmenclip nach einem der Ansprüche 1 bis 5, **dadurch gekennzeichnet, daß** die Klemmleisten (12, 13) im Verbindungsbereich zu den Armen (2, 3) schräg zur Mittelebene des Aneurysmenclips (1) verlaufen.

9. Aneurysmenclip nach einem der voranstehenden Ansprüche, **dadurch gekennzeichnet, daß** der ausgebauchte Abschnitt (9) im wesentlichen kreisförmig ausgebildet ist.

10. Aneurysmenclip nach einem der Ansprüche 1 bis 8, **dadurch gekennzeichnet, daß** der ausgebauchte Abschnitt (9) oval ausgebildet ist.

11. Aneurysmenclip nach einem der voranstehenden Ansprüche, **dadurch gekennzeichnet, daß** die Klemmleisten (12, 13) eine Länge aufweisen, die zwischen dem Zweifachen bis Zehnfachen der Breite der Arme (2, 3) in dem ausgebauchten Abschnitt (9) liegt.

12. Aneurysmenclip nach einem der voranstehenden Ansprüche, **dadurch gekennzeichnet, daß** die beiden Arme (2, 3) über einen federnden Verbindungsabschnitt (17) miteinander verbunden sind.

13. Aneurysmenclip nach einem der Ansprüche 1 bis 11, **dadurch gekennzeichnet, daß** die beiden Arme (2, 3) verschwenkbar miteinander verbunden sind und durch eine separate Feder (5) gegeneinander gespannt werden.

14. Aneurysmenclip nach Anspruch 13, **dadurch gekennzeichnet, daß** die Feder (5) selbst eine Lagerwelle für die Schwenklagerung der beiden Arme (2, 3) bildet.

15. Aneurysmenclip nach einem der voranstehenden Ansprüche, **dadurch gekennzeichnet, daß** die verschwenkbaren Arme (2, 3) in ihrem an die Klemmleisten (12, 13) anschließenden Bereich eine Durchbrechung (21) aufweisen.

16. Aneurysmenclip nach einem der voranstehenden Ansprüche, **dadurch gekennzeichnet, daß** die Arme (2, 3) in ihrem an die Klemmleisten (12, 13) anschließenden Bereich zu den Klemmleisten (12, 13) hin breiter werden.

## Revendications

1. Clip d'anévrisme (1) comportant deux bras (2, 3) qui pivotent de manière élastique l'un vis-à-vis de l'autre et portent à leur extrémité libre des barres de serrage (12, 13) serrées l'une contre l'autre de manière élastique par la force de rappel des bras (2, 3), les parties (9) incurvées vers l'extérieur des bras (2,3) étant adjacentes aux barres de serrage (12, 13) et formant ensemble un espace de réception pour un vaisseau (11), **caractérisé en ce que** les barres de serrage (12, 13) dépassent des bras (2, 3) des deux côtés et font ainsi saillie latéralement des deux côtés sur la largeur des bras (2, 3) dans les parties incurvées vers l'extérieur (9).

2. Clip d'anévrisme selon la revendication 1, **caractérisé en ce que** les barres de serrage (12, 13) sont rectilignes dans leur direction longitudinale.

3. Clip d'anévrisme selon la revendication 1, **caractérisé en ce que** les barres de serrage (12, 13) sont incurvées dans leur direction longitudinale.

4. Clip d'anévrisme selon la revendication 3, **caractérisé en ce que** les bras (2, 3) sont placés du côté convexe des barres de serrage (12, 13).

5. Clip d'anévrisme selon la revendication 3, **caractérisé en ce que** les bras (2, 3) sont placés du côté concave des barres de serrage (12, 13).

6. Clip d'anévrisme selon l'une quelconque des revendications qui précèdent, **caractérisé en ce que** les barres de serrage (12, 13) sont symétriques par rapport au plan central du clip d'anévrisme (1).

7. Clip d'anévrisme selon l'une quelconque des revendications qui précèdent, **caractérisé en ce que**, dans la zone de liaison aux bras (2,3), les barres de serrage (12, 13) s'étendent à un angle de 90° par rapport au plan central du clip d'anévrisme (1).

8. Clip d'anévrisme selon l'une quelconque des revendications 1 à 5, **caractérisé en ce que**, dans la zone de liaison aux bras (2, 3), les barres de serrage (12, 13) s'étendent avec une inclinaison par rapport au plan central du clip d'anévrisme (1).

9. Clip d'anévrisme selon l'une quelconque des revendications qui précèdent, **caractérisé en ce que** la partie incurvée vers l'extérieur (9) est de forme essentiellement circulaire.

10. Clip d'anévrisme selon l'une quelconque des revendications 1 à 8, **caractérisé en ce que** la partie incurvée vers l'extérieur (9) est de forme ovale.

11. Clip d'anévrisme selon l'une quelconque des revendications qui précèdent, **caractérisé en ce que** les barres de serrage (12, 13) ont une longueur comprise entre deux et dix fois la largeur des bras (2, 3) dans la partie incurvée vers l'extérieur (9).

12. Clip d'anévrisme selon l'une quelconque des revendications qui précèdent, **caractérisé en ce que** les deux bras (2, 3) sont reliés entre eux par une section de liaison élastique (17).

13. Clip d'anévrisme selon l'une quelconque des revendications 1 à 11, **caractérisé en ce que** les deux bras (2, 3) sont reliés entre eux de manière à pivoter et sont serrés l'un contre l'autre par un ressort séparé (5).

14. Clip d'anévrisme selon la revendication 13, **caractérisé en ce que** le ressort (5) lui-même forme un axe de support pour le pivot des deux bras (2, 3).

15. Clip d'anévrisme selon l'une quelconque des revendications qui précèdent, **caractérisé en ce que** les bras pivotants (2, 3) présentent une ouverture (21) dans la zone où ils sont adjacents aux barres de serrage (12, 13).

16. Clip d'anévrisme selon l'une quelconque des revendications qui précèdent, **caractérisé en ce que** les bras (2, 3) s'élargissent vers les barres de serrage (12, 13) dans la zone où ils sont adjacents aux barres de serrage (12, 13).
